# EUROPEAN PATENT SPECIFICATION

(11) **EP 4 493 531 B1**
(45) Date of publication and mention of the grant of the patent: **06.05.2026**
(21) Application number: 23712180.1
(22) Date of filing: 14.03.2023
(51) Int. Cl.: C07C 29/149, C07C 31/20

(54) **PROCESS FOR THE PRODUCTION OF ETHYLENE GLYCOL**
VERFAHREN ZUR HERSTELLUNG VON ETHYLENGLYCOL
PROCÉDÉ DE PRODUCTION DU GLYCOL D'ÉTHYLÈNE

(30) Priority: 15.03.2022 EP 22162189
(43) Date of publication of application: 22.01.2025
(73) Proprietor: Avantium Knowledge Centre B.V., 1014 BV Amsterdam (NL)
(72) Inventor: GRUTER, Gerardus Johannes Maria, 1014 BV Amsterdam (NL); SCHULER, Eric, 1012 WX Amsterdam (NL); RAVEENDRAN, Shiju Nirappurackal, 1012 WX Amsterdam (NL)
(74) Representative: Avantium Intellectual Property
(86) International application number: PCT/EP2023/056412
(87) International publication number: WO 2023/174906

(56) References cited:
- WO-A1-2017/134139
- SANTOS JACKSON H S ET AL: "Selective hydrogenation of oxalic acid to glycolic acid and ethylene glycol with a ruthenium catalyst", REACTION KINETICS, MECHANISMS AND CATALYSIS, SPRINGER INTERNATIONAL PUBLISHING, CHAM, vol. 131, no. 1, 5 September 2020 (2020-09-05), pages 139 - 151, XP037255433, ISSN: 1878-5190, [retrieved on 20200905], DOI: 10.1007/S11144-020-01843-3

## Description

### FIELD OF THE INVENTION

The present invention relates to a process for the production of ethylene glycol comprising hydrogenation of oxalic acid.

### BACKGROUND OF THE INVENTION

In view of climate change issues, lots of initiatives are developing to decrease the amount of greenhouse gases in the atmosphere. The focus on capturing CO₂ is increasing, wherein CO₂ is captured mostly from large point sources, such as chemical or power plants, industries with significant CO₂ emissions (such as steelmaking), natural gas processing, the production of hydrogen from fossil fuels, etcetera. After capture, the next steps are CO₂ storage (CCS) and/or utilization (CCU). The significant difference between storage and utilization is that CCS technologies store CO₂ underground so that it cannot re-enter the atmosphere, whereas CCU technologies use CO₂ to convert it to more valuable products, such as plastics or biofuels. Notably, while today the traditional fossil sources of energy production can be replaced by net-zero emission methods (at least in theory), this is not the case for the production of materials, like plastics or concrete. Thus, using CO₂ for the production of materials will be vital for ensuring a lower impact on natural resources and is one of the few options for realizing negative emissions (provided that at least non-fossil CO₂ and renewable energy is used).

For example, CO₂ can be used to produce oxalic acid (HOOCCOOH), and in turn oxalic acid can be hydrogenated to produce for example glycolic acid (HOCH₂COOH; GA) and mono ethylene glycol (HOCH₂CH₂OH; MEG), which are valuable chemicals for a number of different applications, such as for the production of plastics, and for GA as a preservative in food processing, as a skin care agent in cosmetics, and more.

However, although hydrogenation of carboxylic acids is an important organic reaction for the synthesis of useful and valuable chemicals, it is a chemically difficult reaction due to the low reactivity of the carboxy group and the acidic property. This requires for example rational design of catalysts. See: Tamura, M. et al. Recent Developments of Heterogeneous Catalysts for Hydrogenation of Carboxylic Acids to Their Corresponding Alcohols. Asian J. Org. Chem. 2020, 9 (2), 126-143.

Thus, the direct reduction of carboxylic acids requires severe conditions. However, high temperatures cannot be used, as oxalic acid starts to decompose above 130°C, leading to CO₂ and formate, and further to methane under hydrogenation conditions. As a result, there are only a few disclosures in the art about this particular reaction.

In 1955, Carnahan et al. (J.Am.Chem.Soc., 1955, 77 (14), 3766-3768, DOI:10.1021/ja01619a025) reported that glycolic acid could effectively be reduced to mono ethylene glycol above 145°C while using a ruthenium dioxide or ruthenium-on-carbon catalyst. However, very high pressures (650-710 atm., 700-775 atm.) were needed to reach a yield of 80% or higher. They also reported conversion of oxalic acid dihydrate (no solvent) at temperatures between 94 and 170°C using a ruthenium oxide catalyst, and equally high pressures (630-990 atm.) and long reaction times (10.5 hours), to produce a yield of only 47 % of mono ethylene glycol.

The catalytic hydrogenation of an aqueous solution of oxalic acid by a ruthenium-carbon catalyst was reported by Santos et al. (Reaction Kinetics, Mechanism and Catalysis (2020) 131:139-151). They investigated in batch mode the reduction of oxalic acid in the presence of a 5 wt.% Ru/C microporous catalyst in a slurry reactor, at a pressure of 80 bar and a temperature range of 120-150°C, under continuous gas flow, with an operation time of 7 hours. They observed the formation of glycolic acid, acetic acid, ethylene glycol, and volatile compounds. Conversions of oxalic acid of above 90% were reached, however with highest operating selectivity of only 16% (at 130 °C) for ethylene glycol, further a selectivity of 63% (at 120 °C) for glycolic acid and 87% (at 150 °C) for volatile products, respectively.

WO2017134139 discloses a method of preparing glycolic acid and/or ethylene glycol, the method at least comprising the steps of: (a) providing an aqueous oxalic acid containing stream having a molar ratio of water/oxalic acid of above 5.0; (b) subjecting the aqueous oxalic acid containing stream provided in step (a) to hydrogenation in the presence of a hydrogenation metal catalyst and hydrogen, thereby obtaining a glycolic acid containing stream; and (c) optionally subjecting the glycolic acid containing stream obtained in step (b) to hydrogenation in the presence of a hydrogenation metal catalyst and hydrogen, thereby obtaining an ethylene glycol (HOCH₂CH₂OH) containing stream. All the examples of WO2017134139 work in batch mode. The highest selectivity for ethylene glycol reported in the batch process of WO2017134139 is 70% at a conversion of oxalic acid of 99%, produced after a reaction time of in total 18 hours at 90 - 120°C (subsequently 6 hours at 90°C and 12 hours at 120°C), at H₂ pressure of 100-120 bar.

From the Santos publication it can be learned that at relatively high temperature (130 °C) and long operation time (7 hours) the conversion of oxalic acid is relatively high (93%), however producing only 16% of mono ethylene glycol. On the other hand, WO2017134139 teaches that at slightly lower temperature (90 - 120°C) and a much longer reaction time of 18 hours, the selectivity towards mono ethylene glycol is significantly higher (70%) at almost complete conversion of oxalic acid (99%). Even though a selectivity of 70% of mono ethylene glycol is reached, in both these documents, a significant remainder of material is reported for the processes, notably containing for example glycolic acid, acetic acid, ethanol, volatiles (methane, ethane, CO, CO₂), and some starting material (oxalic acid).

Other known routes from oxalic acid to mono ethylene glycol require multiple steps proceding via oxalic acid di-esters as intermediates.

Thus, although it was known that mono ethylene glycol can be prepared by reduction of oxalic acid, commercially attractive conditions to effectively and selectively produce mono ethylene glycol have not yet been reported.

There is still a need for a commercially attractive process for the conversion of oxalic acid into mono ethylene glycol featuring both high conversion of the oxalic acid starting material and a high yield of the desired mono ethylene glycol product. It would be particularly advantageous to provide an industrially applicable, continuous process for the hydrogenation of oxalic acid to selectively produce mono ethylene glycol.

### SUMMARY OF THE INVENTION

Accordingly, the present invention provides a for the production of mono ethylene glycol comprising subjecting an aqueous oxalic acid solution to a hydrogenation reaction in the presence of hydrogen and a metal containing hydrogenation catalyst,
wherein the process is a continuous flow process in a fixed bed reactor;
wherein the aqueous oxalic acid solution and a hydrogen gas stream are fed to the fixed bed reactor;
wherein the reactor comprises a hydrogenation catalyst bed, the catalyst being a supported metal containing hydrogenation catalyst with a total metal loading of from equal to and higher than 2.0 wt % up to equal to and lower than 20.0 wt %; and
wherein the hydrogenation reaction is performed at a temperature selected from the range of from equal to and higher than 95°C up to equal to and lower than 125°C, at a hydrogen pressure selected from the range of from equal to and higher than 10 bar H₂ up to equal to and lower than 150 bar H₂, at a residence time of equal to or longer than 5 minutes up to equal to or lower than 2 hours;
to produce mono ethylene glycol with high selectivity of 80% and higher, at a conversion of oxalic acid of 90% to 100%.

According to the present continuous flow process, high conversion of oxalic acid and in particular high selectivity for mono ethylene glycol were found at short reaction times / residence times (i.e. commercially attractive time frame).

Advantageously, the novel process opens opportunities for efficient industrial production of mono ethylene glycol from oxalic acid.

### DETAILED DESCRIPTION OF THE INVENTION

The present invention relates to a continuous flow process for the production of mono ethylene glycol from oxalic acid. Continuous flow means the process can run uninterrupted and frequent feedstock charging is not needed, as opposed to batch processes. Until now, only a few prior art publications mention the production of mono ethylene glycol from oxalic acid, and those publications only specifically describe the process in batch mode. A "batch process" refers to a process that involves a sequence of steps followed in a specific order, and which process has a beginning and an end. Generally, continuous flow process refers to a process defined by a flow of reactants and products (for every step) wherein the process runs for a longer period of time while continuously feeding fresh reactants and continuously removing the product. Batch processing usually requires more energy, costs more money, and takes longer, but it is often considered the safest and most manageable way to process certain compounds. Continuous processing, though efficient and cost-effective, is not always suitable for any chemical reaction.

The presently disclosed continuous flow process is performed in a fixed bed reactor (also called packed bed reactor), which can be any fixed bed reactor known in the art, for example including a reactor being a single cylindrical tube, but also a multitubular reactor, properly filled / packed with (a) suitable catalyst(s). The catalyst bed, often in the form of pellets, is placed in such a way that it does not move with respect to the reactor itself. The reactants, including reactant gases, uniformly flow over the fixed catalyst bed. A preferred type of fixed bed reactor for performing the present process is a trickle bed reactor. Trickle bed reactors comprise a family of reactors in which gas phase reactants react with liquid phase reactants while flowing in downward direction (toward the direction of gravity) over a bed of solid catalyst particles. The gas phase flow may be both in upward or downward direction depending on the type of application. Preferably, in the present process, the gas phase reactant (hydrogen gas) flows in downward direction, together with the liquid phase reactant (aqueous oxalic acid solution).

The aqueous oxalic acid solution advantageously comprises from equal to or higher than 1.0 weight % to equal to or lower than 40 weight % of oxalic acid, preferably equal to or higher than 2.5 weight %, more preferably equal to or higher than 5.0 weight % to equal to or lower than 20 weight % of oxalic acid.

The selected temperature of the oxalic acid aqueous solution in the feed ("feed temperature") generally is the same temperature as the temperature at which the hydrogenation reaction is performed.

To increase the solubility of oxalic acid in water, certain additives may be used in the aqueous oxalic acid solution, for example alcohols, ethers, etcetera, which are inert under the reaction circumstances. The presence of such additives in the aqueous solution thus has an effect on the saturation, which means that for example a 100% saturated solution comprises more oxalic acid per liter than in the absence of those additives.

Suitably, the aqueous oxalic acid solution that is used as feed may be a recycle stream from the hydrogenation reaction or it may comprise a recycle stream from the hydrogenation reaction. Said recycle stream can therefore comprise one or more hydrogenation (side) products, such as glycolic acid, ethylene glycol, glyoxylic acid, acetic acid, etc.. Preferably, the aqueous oxalic acid solution that is used as feed comprises at most 20 weight % in total of such hydrogenation (side) products.

In a preferred embodiment of the presently claimed process, the aqueous oxalic acid solution used as feed does not comprise potassium in any form. The presence of potassium is considered to be poisonous to the activity of catalyst.

Suitably, the catalyst in the fixed bed is a supported metal containing hydrogenation catalyst with a total metal loading of from equal to and higher than 2.0 wt % up to equal to and lower than 20.0 wt %, and preferably from equal to and higher than 3.0 wt % up to equal to and lower than 15.0 wt %, and particularly up to equal to and lower than 12.0 wt%.

The hydrogenation catalyst preferably contains one or more metals selected from group A metals: platinum, nickel, copper, ruthenium, rhodium and iridium, and preferably ruthenium. Further, the hydrogenation catalyst optionally also contains one other metal selected from group B metals: tin, bismuth, palladium, rhenium, gold, and antimony. Preferably, the hydrogenation catalyst contains ruthenium (group A metal), and preferably one group B metal.

A highly preferred hydrogenation catalyst contains ruthenium and tin. Such ruthenium-tin catalyst can advantageously be used to suppress side product formation. The molar ratio of ruthenium and tin in the catalyst from 10:1 to 1:10, preferably 5:1 to 1:5, more preferably 5:2 to 1:4.

A further preferred catalyst for use in the process is a trimetallic catalyst containing ruthenium, platinum and tin. See e.g. S. Taniguchi et al. / Applied Catalysis A: General 397 (2011) 171-173*.* The amounts and molar ratio of ruthenium and tin in the trimetallic catalyst are selected as described above. The amount of platinum is preferably 1 to 5 weight %, more preferably 1.5 to 3 weight % relative to the catalyst support. A highly preferred catalyst for optimal production of mono ethylene glycol from oxalic acid is Ru₁Sn_{0.85}Pt_{0.2}/C, the catalyst having a metal loading 5 wt % of ruthenium, 5 wt % of tin and 2 wt % of platinum. The oxalic acid conversion rate is increased when platinum is also present in the catalyst.

Advantageously, no chloride is present in the catalyst used in the present process, which is considered important for catalyst stability.

Preferably, the catalyst used in the process according to the present disclosure is calcined (pre-treated) prior to use at a temperature selected from 200°C to 450°C.

The catalyst support can be selected from any support material that does not interfere with the current hydrogenation reaction. It was found that an aluminum oxide supported ruthenium catalyst hardly leads to conversion of oxalic acid into mono ethylene glycol. Preferably, the hydrogenation catalyst is supported on a carrier selected from carbon, silicon carbide, MAX-Phase (Ti₂Al₂C),TiO₂ and ZrO₂ , more preferably carbon or MAX-Phase.

According to the present invention, reaction conditions were found that are very useful for a continuous flow process for the selective production of mono ethylene glycol with high oxalic acid conversion. At temperatures from equal to and higher than 95°C up to equal to and lower than 125°C, in particular from equal to and higher than 105°C up to equal to and lower than 125°C, and within a commercially attractive time frame, advantageously selectivity for mono ethylene glycol of particularly at least 85% could be obtained at a conversion of oxalic acid of from equal to and higher than 90 % up to and including 100 %.

The commercially attractive time frame in which the present continuous flow process takes place, is expressed in terms of "residence time". The term "residence time" defines the average length of time that the feed, or specifically a molecule in the feed, remains inside the reactor, meaning herein inside the part that contains the catalyst bed. According to the process of the present invention, a residence time of equal to or longer than 5 minutes up to equal to or less than 2 hours is required. Preferably, the residence time is equal to or longer than 10 minutes up to equal to or less than 1 hour. For flow reactors suitable for the present hydrogenation reaction, the residence time may be varied by varying factors like the reactor volume, the feed flow rate (both of the oxalic acid solution and of the hydrogen gas), the length of the catalyst bed, etc.. When compared to continuous flow processes, in a batch process, the residence time can be related to reaction time, i.e. how long a container is held at a specific temperature in the batch process.

In the process of the invention, it was found that increasing the feed flow does not affect the selectivity towards the product. If the feed flow is very high, resulting in very short residence times, only the conversion of oxalic acid does not run until completion, but still selectively mono ethylene glycol is formed. The person skilled in the art will understand how to balance the feed flow at a certain temperature against the most appropriate residence time. At a relatively high temperature, shorter residence times (and thus higher feed flow) will be required to maximize both the conversion of oxalic acid and selectivity towards the desired product.

The current process is performed at a hydrogen pressure selected from the range of from equal to and higher than 10 bar H₂ up to equal to and lower than 150 bar H₂, preferably at 20 to 100 bar H₂.

The product stream of the continuous flow process according to the present disclosure may be continuously removed from the process and directly subjected to further processing if deemed necessary, but also the product stream may be (partly) recycled before the desired glycolic acid is separated from the solution.

Oxalic acid is a corrosive substance. Stainless steel reactors are therefore not ideal for the present process. Advantageously, therefore, the present hydrogenation reaction is performed in a reactor with non-metallic or inert liners, such as Teflon, glass, PVC, titanium or Hastelloy.

### BRIEF DESCRIPTION OF THE DRAWING

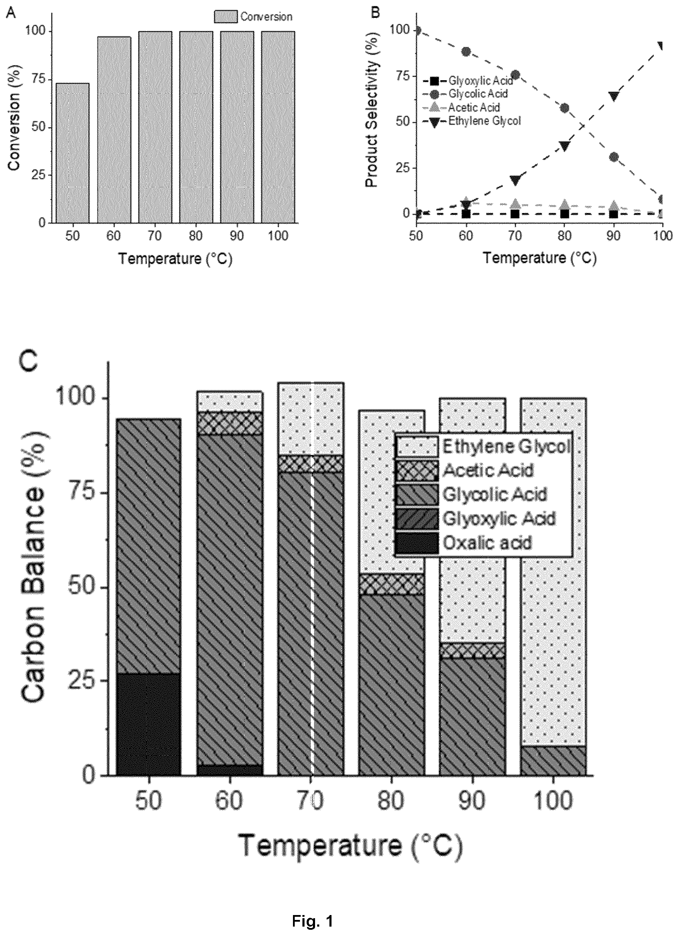

Fig. 1. Results of SFU experiment of oxalic acid reduction for different temperatures. Results of different experiments were combined into one graph. Conversion (A), Selectivity (B) and Carbon Balance (C) data as obtained by liquid chromatography (LC). Conditions during reactions: Temperature = 50-100°C, Pressure = 60 bar, Feed = Oxalic Acid (5 wt.%) in water, Flow of Feed = 0.1 ml min⁻¹, Flow of Gas (H₂) = 200 ml min⁻¹. This reaction used commercial Ru/C catalyst (Johnson Mattey 9.76 wt.% Ru/C, 0.5 wt.% moisture). Carbon balance has been corrected.

The invention is further illustrated by the following non-limiting examples.

### EXAMPLES

### List of abbreviations

(M)EG = (mono) ethylene glycol
OA = oxalic acid
SFU = Single Flow Unit

### Experimental

### Materials and reagents

Oxalic acid (C₂H₂O₄) was obtained from Sigma-Aldrich^{®}), dried and stored in a dry environment. All water used during this work was filtered using a Millipore system.

The catalyst that was used was 9.76 wt.% Ruthenium on Carbon, supplied by Johnson Matthey (ID: 110005, LOT M17160). The catalyst contained 0.5% moisture.

### Catalyst reduction and preparation

Catalyst extrudates were crushed with a ceramic mortar and sieved to obtain a mesh size between 105-200 µm. Catalyst particles were transferred to a ceramic crucible and placed in a tubular furnace for reduction. In the furnace, the particles were treated with a gas mixture of 7% H₂ in N₂ with a flow rate of 100 ml min⁻¹. The temperature inside the furnace was increased with a ramp of 10°C min⁻¹ until it reached 300-450°C. This temperature was kept constant for 180 minutes. Upon completion of the reduction procedure, the temperature was slowly decreased, and the 7% H₂/N₂ mixture was flushed out with a flow of pure nitrogen.

### Single Flow Unit (SFU) Components

Flow chemistry experiments were performed in a trickle bed reactor system. This unit can be divided into different sub-sections. The gas flow was controlled by 2 mass flow controllers (MFC's) for both nitrogen and hydrogen. The Nitrogen MFC was capable of a flow up to 1000 ml min⁻¹, whereas the Hydrogen MFC was able to go up to 200 ml min⁻¹. The pressure in the reactor was controlled by a pressure indicator, which puts pressure on a back pressure regulator located after the reactor to regulate the pressure inside the system. The feed section consisted of a Jasco HPLC pump. The liquid feed flow rate range was between 0.1 and 5 ml min⁻¹, which was transferred separately from the gas flow to the reactor.

The reactor used for the experiments was a 30 cm long reactor tube, with a diameter of 4.6 mm. The reactor volume of this reactor was 5 ml, of which 1.82 ml was within the isothermal zone of the system and therefore allowed to be loaded with a catalyst.

This system had space for 8 separate effluent sample vials, which collected effluent that had passed the reactor and directed to the sample vial through a selector valve system. The sample vials had 2 needles inserted, one input needle from the selector valve and one output needle to release pressure or overflow feed towards a "Flush" jerry can. The selector valve itself had 16 channels, of which 8 channels led towards the sample vials and 8 channels that went through a manifold towards a "Waste" jerry can.

The system was provided with a heating and stirring system for the feed solution which allowed for heating and stirring of the feed solution to create a more homogeneously distributed feed solution.

### Reactor materials

As oxalic acid is a good complexing agent, it can potentially leach metals from the reactor components or the catalyst. To get an idea for leaching, we analysed the reaction solution after reaction of 25 wt% OA in water in a Hastelloy reactor for 2 hours at 65 °C with ICP-OES. We observed strong leaching of chrome, iron, and nickel but no leaching of ruthenium from the catalyst. To study the effect of these metals in the solutions we added three times the amount found in the leaching experiments of Cr, Fe, Ni to the starting solution. We noticed inhibition of the initial reaction rate by 10-15 %. To prevent leaching, we tested non-metallic liners for the Hastelloy reactor and avoided any metal parts contacting the reaction solution. We compared the performance of the reactor with and without Teflon and glass liners and found better catalyst stability with liners. We performed the reaction six times consecutively with the same solvent to amplify the potential effect of leaching. For each of the six reactions, we added fresh reactant solution but kept the catalyst in the reactor. The Teflon liners worked best to preserve the catalytic activity as the high over-reduction to ethylene glycol. Metal ions from the reactor corrosion at ppm levels (Ni, Cr, Mo) might be inhibiting the catalyst performance and only Teflon liners provide adequate protection.

### Reactor Loading

Single flow reactions used a 30 cm long stainless steel reactor. Equilibration experiments were performed before this study on this reactor to determine that the isothermal zone lies between 9 and 20 cm from the bottom up. The internal diameter of the reactor was 4.6 mm, which corresponds to a reactor volume of 5 ml (or 1.82 ml for the isothermal zone). For each reactor loading, the maximum volume (1.82 ml) of catalyst was used to fill up the isothermal zone of the reactor. The catalyst was reduced ex-situ at 350 °C in a hydrogen atmosphere, packed into the reactor bed and pre-reduced in-situ in the flow reactor at 200 °C in a hydrogen atmosphere. The remaining part of the reactor was filled up with layers of Silica Carbide (SiC) and Quartz wool.

### Reactor attaching

The stainless steel reactor was equipped with Swagelok fittings. Through standard Swagelok attaching procedure, the reactor was attached to the SFU. Swagelok fittings are the choice of fitting here because of their ability to hold high pressures while remaining leak-free.

### Analysis

Liquid chromatography was performed as follows. Samples were prepared by diluting stock solutions to concentrations of 0.67 mg stock mL⁻¹ in demineralized water. An Agilent Technologies 1260 Infinity II was used to measure the concentration of oxalic acid, glycolic acid, glyoxylic acid, acetic acid, and ethylene glycol. To confirm the results by a second method we used quantitative liquid phase IR measurements in random order. The results for both methods matched.

### Examples

The system used for these reactions is a trickle-bed reactor system, which allows unlimited continuous operation and automated collection of 8 samples per experiment without interruption. Jasco HPLC pumps create a reactant feed flow, while mass flow controllers (MFCs) control gas (H₂ or N₂) flow towards the reactor. On top of the reactor, both the feed and gas flow merge, which causes a downward movement of the liquid and co-current movement of the gas over the packed catalyst bed.

### Control experiments

(1) Absence of catalyst - Control experiments were performed to exclude any possible reaction of the trickle bed reactor with the reactant feed. These control experiments employed similarly loaded reactors as described in the section "Reactor Loading". For the control experiments, the catalyst in the isothermal zone of the system was replaced with silica carbide. Different reactant feed flow rates (5 to 0.1 ml min⁻¹) were examined.
   Results: Near-zero conversion of oxalic acid was observed towards observable reaction products during these reactions. The observed marginal conversion lies within the margin of error of the analysis method. Liquid chromatography showed only oxalic acid. In conclusion, in the employed system no oxalic acid converts in the absence of any catalyst.
(2) No hydrogen - The influence of hydrogen presence was investigated. The reactor according to the procedure described in the section "Reactor Loading". For this experiment, a commercial catalyst provided by Johnson Mattey with the following characteristics (9.76 wt.% Ru/C, 0.5 wt.% moisture) was loaded within the isothermal zone of the reactor. Instead of a hydrogen flow, different flows of nitrogen were examined. The reaction conditions during this experiment were similar to conditions of the actual experiments described herein that did yield glycolic acid, the only exception being a nitrogen flow instead of hydrogen.
   Results: Similar to the results of the control experiment in the absence of a catalyst, this experiment yielded no reaction products. The conversions were so marginal that they are within the margin of error of the LC analysis, and no conclusions regarding any possible conversion can be derived from these data.

From the controls and pre-experiments, it was concluded that the conversion of oxalic acid requires the presence of a catalyst and pressurized hydrogen.

### Example 1

The reduction of oxalic acid was performed at different temperatures in the SFU. For each of these reactions, catalyst particles (size: 105-200 µm) of the same commercial catalyst from Jonhson Mattey (9.76 wt.% Ru/C, 0.5 wt.% moisture) were loaded in the isothermal zone of the reactor. For the data shown in Fig 1 results from different temperature-effect experiments were combined. For these experiments a reactant feed flow rate of 0.1 ml min-1 was used, which corresponds to a residence time of 1092 seconds for full liquid conditions (18.2 minutes). The hydrogen flow rate was kept constant at 200 ml min-1, which means a constant gas/feed ratio of 2000 was maintained during the experiments. Pressure = 60 bar. Feed = Oxalic Acid (5 wt.%) in water. The feed was not heated or stirred during the reactions, which caused a slightly heterogeneous distribution of oxalic acid in the reactant feed bottle, resulting in small concentration differences. Because of this inaccuracy, the carbon balance has been corrected to 96% using a correction factor, to give a more accurate depiction of the real carbon balance. Fig 1. shows that the conversion of oxalic acid increases with temperature from 50 - 70 °C, reaching 100%, and then remains constant with further rise in temperature. Initially, oxalic acid is converted to glycolic acid only (Fig. 1 B, at 50 °C). At higher temperatures the reaction rate increases and ethylene glycol is formed from glycolic acid, and is produced selectively, particularly above 95 °C.

## Claims

1. A process for the production of mono ethylene glycol comprising subjecting an aqueous oxalic acid solution to a hydrogenation reaction in the presence of hydrogen and a metal containing hydrogenation catalyst,
wherein the process is a continuous flow process in a fixed bed reactor;
wherein the aqueous oxalic acid solution and a hydrogen gas stream are fed to the fixed bed reactor;
wherein the reactor comprises a hydrogenation catalyst bed, the catalyst being a supported metal containing hydrogenation catalyst with a total metal loading of from equal to and higher than 2.0 wt % up to equal to and lower than 20.0 wt %; and
wherein the hydrogenation reaction is performed at a temperature selected from the range of from equal to and higher than 95°C up to equal to and lower than 125°C, at a hydrogen pressure selected from the range of from equal to and higher than 10 bar H₂ up to equal to and lower than 150 bar H₂, at a residence time of equal to or longer than 5 minutes up to equal to or lower than 2 hours;
to produce mono ethylene glycol with high selectivity of 80% and higher, at a conversion of oxalic acid of 90% to 100%.

2. The process of claim 1, wherein the reactor is a trickle bed reactor.

3. The process of claim 1 or 2, wherein the aqueous oxalic acid solution comprises from equal to or higher than 1.0 weight % to equal to or lower than 40 weight % of oxalic acid.

4. The process of any one of claims 1 to 3, wherein the hydrogenation catalyst contains one or more metals selected from group A metals: platinum, nickel, copper, ruthenium, rhodium and iridium, and optionally one other metal selected from group B metals: tin, bismuth, palladium, rhenium, gold, and antimony.

5. The process of any one of claims 1 to 4, wherein the hydrogenation catalyst contains ruthenium as group A metal, and preferably one group B metal.

6. The process of claim 5, wherein the hydrogenation catalyst contains ruthenium and tin.

7. The process of claim 6, wherein the molar ratio of ruthenium to tin in the catalyst is from 10:1 to 1:10, preferably 5:1 to 1:5, more preferably 5:2 to 1:4.

8. The process of any one of claims 1 to 7, wherein the catalyst is a trimetallic catalyst containing ruthenium, tin and platinum.

9. The process of any one of claims 1 to 8, wherein the hydrogenation catalyst is supported on a carrier selected from carbon, silicon carbide, MAX-Phase (Ti₂Al₂C), TiO₂ andZrO₂ , preferably carbon or MAX-Phase.

10. The process of any one of claims 1 to 9, wherein the hydrogenation reaction is performed in a reactor with non-metallic or inert liners, preferably selected from Teflon, glass, PVC, titanium and Hastelloy.

## Patentansprüche

1. Verfahren zur Herstellung von Monoethylenglykol, umfassend Durchführen einer Hydrierungsreaktion in Gegenwart von Wasserstoff und einem Metall, das einen Hydrierungskatalysator enthält, an einer wässrigen Oxalsäurelösung,
wobei das Verfahren ein kontinuierliches Durchflussverfahren in einem Festbettreaktor ist;
wobei die wässrige Oxalsäurelösung und ein Wasserstoffgasstrom dem Festbettreaktor zugeführt werden;
wobei der Reaktor ein Bett für den Hydrierungskatalysator umfasst, wobei der Katalysator ein geträgertes Metall ist, das einen Hydrierungskatalysator mit einer Gesamtmetallladung von gleich und höher als 2,0 Gew.-% bis gleich und niedriger als 20,0 Gew.-% enthält; und
wobei die Hydrierungsreaktion bei einer Temperatur durchgeführt wird, die aus dem Bereich von gleich und höher als 95 °C bis gleich und niedriger als 125 °C ausgewählt ist, bei einem Wasserstoffdruck, der aus dem Bereich von gleich und höher als 10 bar H₂ bis gleich und niedriger als 150 bar H₂ ausgewählt ist, bei einer Verweilzeit von gleich oder länger als 5 Minuten bis gleich oder kürzer als 2 Stunden;
zum Herstellen von Monoethylenglykol mit einer hohen Selektivität von 80 % und höher, bei einer Umwandlung der Oxalsäure von 90 % bis 100 %.

2. Verfahren nach Anspruch 1, wobei der Reaktor ein Rieselbettreaktor ist.

3. Verfahren nach Anspruch 1 oder 2, wobei die wässrige Oxalsäurelösung gleich oder mehr als 1,0 Gewichts-% bis gleich oder weniger als 40 Gewichts-% Oxalsäure umfasst.

4. Verfahren nach einem der Ansprüche 1 bis 3, wobei der Hydrierungskatalysator ein oder mehrere Metalle umfasst, die aus den Metallen der Gruppe A ausgewählt sind: Platin, Nickel, Kupfer, Ruthenium, Rhodium und Iridium, und optional ein anderes Metall, das aus den Metallen der Gruppe B ausgewählt ist: Zinn, Bismut, Palladium, Rhenium, Gold und Antimon.

5. Verfahren nach einem der Ansprüche 1 bis 4, wobei der Hydrierungskatalysator Ruthenium als Metall der Gruppe A und bevorzugt ein Metall der Gruppe B enthält.

6. Verfahren nach Anspruch 5, wobei der Hydrierungskatalysator Ruthenium und Zinn enthält.

7. Verfahren nach Anspruch 6, wobei das Molverhältnis von Ruthenium zu Zinn in dem Katalysator 10:1 bis 1:10 beträgt, bevorzugt 5:1 bis 1:5, bevorzugter 5:2 bis 1:4.

8. Verfahren nach einem der Ansprüche 1 bis 7, wobei der Katalysator ein Katalysator aus drei Metallen ist, der Ruthenium, Zinn und Platin enthält.

9. Verfahren nach einem der Ansprüche 1 bis 8, wobei der Hydrierungskatalysator auf einem Träger getragen wird, der ausgewählt ist aus Kohlenstoff, Siliziumcarbid, MAX-Phase (Ti₂Al₂C), TiO₂ und ZrO₂, bevorzugt Kohlenstoff oder MAX-Phase.

10. Verfahren nach einem der Ansprüche 1 bis 9, wobei die Hydrierungsreaktion in einem Reaktor mit nicht-metallischen oder inerten Auskleidungen durchgeführt wird, bevorzugt ausgewählt aus Teflon, Glas, PVC, Titan und Hastelloy.

## Revendications

1. Processus de production de monoéthylène glycol comprenant la soumission d'une solution aqueuse d'acide oxalique à une réaction d'hydrogénation en présence d'hydrogène et d'un catalyseur d'hydrogénation contenant un métal,
dans lequel le processus est un processus d'écoulement continu dans un réacteur à lit fixe ;
dans lequel la solution aqueuse d'acide oxalique et un flux d'hydrogène gazeux sont alimentés dans le réacteur à lit fixe ;
dans lequel le réacteur comprend un lit de catalyseur d'hydrogénation, le catalyseur étant un catalyseur d'hydrogénation contenant un métal supporté ayant une charge métallique totale allant de 2,0 % ou plus en poids à 20,0 % ou moins en poids ; et
dans lequel la réaction d'hydrogénation est effectuée à une température choisie dans la plage allant de 95 °C ou plus à 125 °C ou moins, à une pression d'hydrogène choisie dans la plage allant de 10 bars H₂ ou plus à 150 bars H₂ ou moins, à un temps de séjour allant de 5 minutes ou plus à 2 heures ou moins ;
pour produire du monoéthylène glycol ayant une sélectivité élevée de 80 % ou plus, à une conversion d'acide oxalique de 90 % à 100 %.

2. Processus selon la revendication 1, dans lequel le réacteur est un réacteur à lit ruisselant.

3. Processus selon la revendication 1 ou 2, dans lequel la solution aqueuse d'acide oxalique comprend de 1,0 % ou plus en poids à 40 % ou moins en poids d'acide oxalique.

4. Processus selon l'une quelconque des revendications 1 à 3, dans lequel le catalyseur d'hydrogénation contient un ou plusieurs métaux choisis parmi le groupe de métaux A : platine, nickel, cuivre, ruthénium, rhodium et iridium, et facultativement un autre métal choisi parmi le groupe de métaux B : étain, bismuth, palladium, rhénium, or et antimoine.

5. Processus selon l'une quelconque des revendications 1 à 4, dans lequel le catalyseur d'hydrogénation contient du ruthénium comme métal du groupe A, et de préférence un métal du groupe B.

6. Processus selon la revendication 5, dans lequel le catalyseur d'hydrogénation contient du ruthénium et de l'étain.

7. Processus selon la revendication 6, dans lequel le rapport molaire du ruthénium à l'étain dans le catalyseur est de 10:1 à 1:10, de préférence de 5:1 à 1:5, plus préférablement de 5:2 à 1:4.

8. Processus selon l'une quelconque des revendications 1 à 7, dans lequel le catalyseur est un catalyseur trimétallique contenant du ruthénium, de l'étain et du platine.

9. Processus selon l'une quelconque des revendications 1 à 8, dans lequel le catalyseur d'hydrogénation est supporté sur un support choisi parmi carbone, carbure de silicium, phase MAX (Ti₂Al₂C), TiO₂ et ZrO₂, de préférence carbone ou phase MAX.

10. Processus selon l'une quelconque des revendications 1 à 9, dans lequel la réaction d'hydrogénation est effectuée dans un réacteur avec des revêtements non métalliques ou inertes, de préférence choisis parmi Téflon, verre, PVC, titane et Hastelloy.
